(19) Europäisches Patentamt
European Patent Office
Office européen des brevets

(11) Publication number : **0 378 707 B1**

(12) # EUROPEAN PATENT SPECIFICATION

(45) Date of publication of patent specification : **20.01.93 Bulletin 93/03**

(51) Int. Cl.[5] : **C07C 323/62,** C07C 323/58, C07C 323/61, C07C 327/34, C07D 307/38, C07D 307/91, C07D 333/34

(21) Application number : **89908282.0**

(22) Date of filing : **05.07.89**

(86) International application number : **PCT/JP89/00672**

(87) International publication number : **WO 90/00543 25.01.90 Gazette 90/03**

(54) **AMINOACETONITRILE DERIVATIVES.**

(30) Priority : **07.07.88 JP 167738/88**

(43) Date of publication of application : **25.07.90 Bulletin 90/30**

(45) Publication of the grant of the patent : **20.01.93 Bulletin 93/03**

(84) Designated Contracting States : **AT BE CH DE FR GB IT LI LU NL SE**

(56) References cited :
**EP-A- 0 135 304**
**EP-A- 0 149 324**

(73) Proprietor : **NIPPON SODA CO., LTD.**
**2-1, Ohtemachi 2-chome**
**Chiyoda-ku, Tokyo 100 (JP)**

(72) Inventor : **TAZAKI, Seiji,Mizushima Plant,Nippon Soda Co., Ltd.,2767-12, Aza Niihama, Shionasu Kojima, Kurashiki-shi,Okayama (JP)**
Inventor : **UMEDA, Nobuhiro,Odawara Research Center Nippon Soda Co., Ltd.,345, Aza Yanagimachi Takada, Odawara-shi,Kanagawa (JP)**
Inventor : **MATSUI, Nobuo,Odawara Research Center Nippon Soda Co., Ltd.,345, Aza Yanagimachi Takada, Odawara-shi,Kanagawa (JP)**
Inventor : **YAGIHARA, Tomio,Mizushima Plant,Nippon Soda Co., Ltd.,2767-12, Aza Niihama, Shionasu Kojima, Kurashiki-shi,Okayama (JP)**
Inventor : **MIKUMA, Katsunori,Mizushima Plant,Nippon Soda Co., Ltd.,2767-12, Aza Niihama, Shionasu Kojima, Kurashiki-shi,Okayama (JP)**

(74) Representative : **de Bruijn, Leendert C. et al**
**Nederlandsch Octrooibureau**
**Scheveningseweg 82 P.O. Box 29720**
**NL-2502 LS Den Haag (NL)**

EP 0 378 707 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to new aminoacetonitrile derivatives where the 2-position of aminoacetonitrile is substituted by alkylthio groups, arylthio groups or heterocyclicthio groups.

The aminoacetonitrile derivatives of this invention can be used as starting materials for the manufacture of pharmaceutical and/or agricultural intermediates such as benzamide derivatives with fungicidal and herbicidal activities as shown in EP-135304.

They can also be used as starting materials for the manufacture of dyes, perfumes, polymers, and so on.

Background Art:

α-substituted aminoacetonitrile derivatives are usually prepared via 3 steps (see EP 135304): aminoacetonitrile, prepared by the reaction of hydrogen cyanide with formaldehyde, followed by treatment with excess of ammonia, is acylated for the protection of amino group, and then halogenated to give α-halo -acylaminoacetonitrile which is allowed to react with nucleophiles such as alcohol, thiol or amine to give an aimed compound. However, α-substituted aminoacetonitrile is not obtained by this method because hydrolysis of a-substituted acylaminoacetonitrile may be difficult.

The purpose of this invention is to provide new and stable aminoacetonitrile derivatives and their salts which can be used as starting materials for various types of organic compounds, and furthermore their inexpensive synthetic methods.

Disclosure of Invention:

This invention relates to an aminoacetonitrile derivative having the formula

$$\underset{\displaystyle NH_2}{RS\overset{}{C}HCN} \qquad (I)$$

wherein R represents (a) an alkyl group which may be substituted by phenyl group(s) (which may be substituted by halogen atom(s) or $C_{1-3}$ alkyl group(s)), $C_{1-3}$ alkoxy-carbonyl group(s), hydroxy group(s), mercapto group(s), halogen atom(s), naphthyl group(s), furyl group(s) or $R_1$NHCO-group(s) (where $R_1$ is a phenyl group which may be substituted by halogen atom(s), an alkyl group which may be substituted by phenyl group(s) or a cycloalkyl group), (b) a phenyl group which may be substituted by halogen atom(s), $C_{1-5}$ alkyl group(s) (which may be substituted by halogen atom(s) or phenyl group(s), cycloalkyl group(s), phenyl group(s) (which may be substituted by halogen atom(s)), phenoxy group(s) (which may be substituted by $C_{1-3}$ alkyl group(s) or haloalkyl group(s)), tetrahydronaphthoxy group(s), $C_{1-3}$ alkoxy group(s) (which may be substituted halogen atom(s)), nitro group(s), amino group(s) or hydroxy group(s), (c) a cycloalkyl group, (d) a $C_{2-4}$ alkenyl group, (e) a naphthyl group which may be substituted by halogen atom(s), (f) a dibenzofuranyl group, (g) a $C_{1-3}$ alkylcarbonyl group, (h) a thienyl group or (i) a fluorenyl group ;
or its salts, and their preparation processes.

The aminoacetonitrile derivative represented by the above general formula (I) can be generally isolated as crystal of salts such as p-toluenesulfonate or hydrochloride.

These salts exist extremely stable, and can be purified by ordinary methods such as recrystallization.

Best Mode for Carrying Out the Invention :

The aminoacetonitrile derivatives having the formula (I) can be manufactured by the following processes

(1) Manufacturing process A

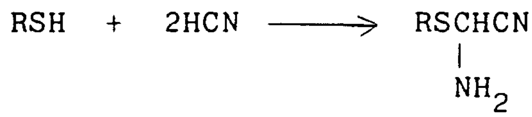

(II) (III) (I)

The reaction is carried out in organic solvents in the presence of a base at 0°C to 40 °C for 30 minutes to several hours, and, after usual work-up, an acid including an organic acids such as oxalic acid, organic sulfonic acids such as p-toluenesulfonic acid and naphthalene sulfonic acid, or inorganic acids such as hydrochloric acid, sulfuric acid or phosphoric acid, is added to the resulting organic layer to isolate an aminoacetonitrile derivative as the salt of the acid used.

An appropriate thiol is used depending on the aimed compound.

Reaction solvents used include hydrocarbons such as benzene, toluene and xylene, nitriles such as acetonitrile, halogenated hydrocarbons such as chloroform and methylene chlorides, esters such as ethyl acetate, and alcohols such as ethanol. These can be used as mixture.

For the base, inorganic basic compounds such as sodium carbonate and organic basic compounds such as triethylamine, diethylamine and diazabicycloundecene (DBU) may be used.

A range of molar concentration (mol) of each component is generally 1 for Compound (II), 2 - 20, preferably 4 - 8 for Compound (III) and 0.1 - 2, preferably 0.2 - 0.6 for the base.

The reaction is usually carried out under the atomospheric pressure, though media used, types of solvents, etc and amount used may differ depending on purpose. The salt of aminoacetonitrile derivatives isolated from the reaction solution is reacted with alkali to easily isolate free intended compounds, which, if necessary, can be purified by column chromatography.

(2) Manufacturing process B

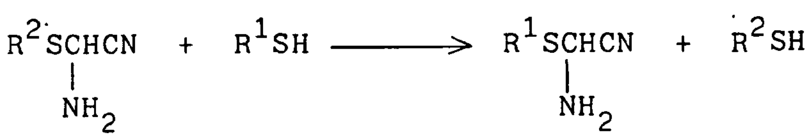

(I)' (II)' (I)" (II)"

wherein $R^1$ represents an (a) alkyl group which may be substituted by phenyl group(s) (which may be substituted by halogen atom(s) or $C_{1-3}$ alkyl group(s)), $C_{1-3}$ alkoxy-carbonyl group(s), hydroxy group(s), mercapto group(s), halogen atom(s), naphthyl group(s), furyl group(s) or $R_1$NHCO- group(s) (where $R_1$ is a phenyl group which may be substituted by halogen atom(s), an alkyl group which may be substituted by phenyl group(s) or a cycloalkyl group), (b) a cycloalkyl group, or (c) a $C_{2-4}$ alkenyl group ;

$R^2$ represents (a) a phenyl group which may be substituted by halogen atom(s), $C_{1-5}$ alkyl group(s) (which may be substituted by halogen atom(s) or phenyl group(s)), cycloalkyl group(s), phenyl group(s) (which may be substituted by halogen atom(s)), phenoxy group(s) (which may be substituted by $C_{13}$ alkyl group(s) or haloalkyl group(s)), tetrahydronaphthoxy group(s), $C_{1-3}$ alkoxy group(s) (which may be substituted halogen atom(s)), nitro group(s), amino group(s) or hydroxy group(s), (1) a naphthyl group which may be substituted by halogen atom(s), (c) a thienyl group or (d) a fluorenyl group.

The reaction is carried out in organic solvents in the presence of a base at 0°C to 80°C for 30 minutes to several hours.

For the organic solvents and the base, the items specified for the manufacturing process A can be used.

After the reaction are completed, the similar procedures operated under the manufacturing process A are carried out to obtain salts or free forms of intended products.

The structure of the product was determined by such means as NMR, IR, Mass spectra and elemental analysis, and, if necessary, by leading to the known compound.

The present invention is described in detail by using examples. The scope of the invention, however, is by no means limited by these examples.

Example 1

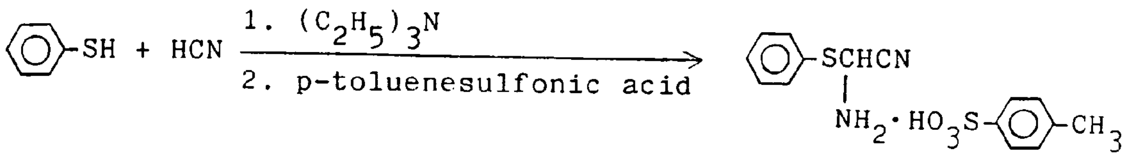


(Compound No. 1)

In a 500ml flask were placed thiophenol (17.4g), triethylamine (8.0g) and benzene (150ml).

Liquid hydrogen cyanide (25.6g) was added to this solution at 5°C and stirring was continued at 20°C for 1.5 hours.

Then p-toluenesulfonic acid monohydrate (30.0g) was added to the reaction mixture and stirring was continued at 0 - 5°C for 10 minutes. The white precipitate was filtered, washed with diethyl ether (20ml) and acetonitrile (20ml) and dried in a vacuum desiccator.

Phenylthioaminoacetonitrile p-toluenesulfonate (38-.0g) was obtained in a yield of 71.5%. m.p. 145 - 146°C.

Example 2

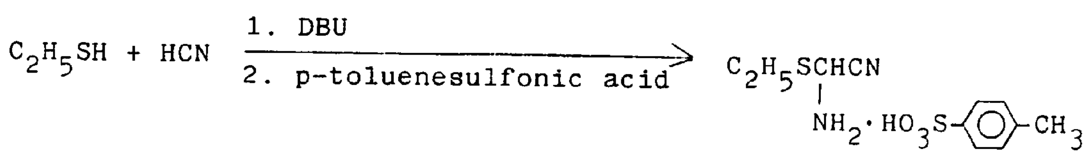


(Compound No. 4)

In a 100ml flask were placed ethanethiol (2.5g), DBU (3.0g) and acetonitrile (40ml).

Liquid hydrogen cyanide (6.5g) was added to this solution at 5°C and stirring was continued at 20°C for 3 hours. The reaction mixture was poured into 10% sodium hydroxide (60g) which was precooled at 5°C in 300ml glass vessel, followed by extracting with chloroform (20ml) twice. The chloroform layer was dried over anhydrous magnesium sulfate.

p-Toluenesulfonic acid monohydrate (7.6g) was added to the dried chloroform layer and stirring was continued at 0 - 5°C for 10 minutes. The white precipitate was filtered, washed with diethyl ether (10ml) and acetonitrile (10ml) dried in a vacuum desiccator.

Ethylthioaminoacetonitrile p-toluenesulfonate (4.9g) was obtained in a yield of 40.0%. m.p. 125 - 126°C.

Example 3

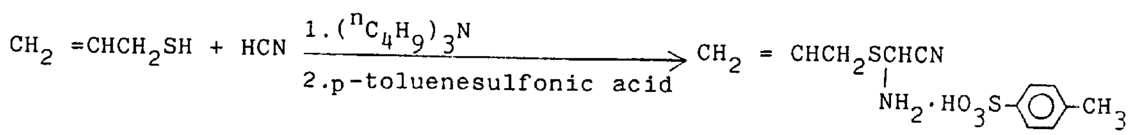


(Compound No. 7)

In a 100ml flask were placed allyl mercaptan (2.2g), tri-n-Butyl amine (1.9g) and dichloromethane (20ml). Liquid hydrogen cyanide (3.3g) was added to this solution at 5°C and stirring was continued at 20°C for 1.5 hours. When worked up as Example 1, allylthioaminoacetonitrile p-toluenesulfonate (3.1g) was obtained in a yield of 48.7%. m.p. 127 - 129°C.

Example 4

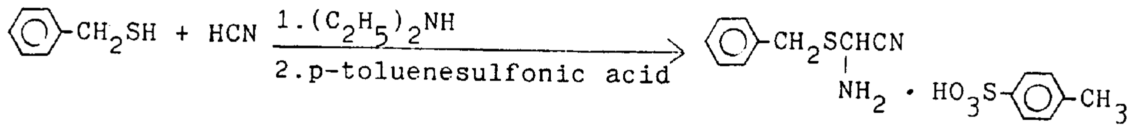


(Compound No. 8)

In a 100ml flask were placed benzyl mercaptan (2.5g), diethyl amine (0.8g) and xylene (20ml).

Liquid hydrogen cyanide (3.3g) was added to this solution at 5°C and stirring was continued at 20°C for 1 hour. When worked up as Example 1, benzylthioaminoacetonitrile p-toluenesulfonate (3.3g) was obtained in a yield of 45.0%. m.p. 143 - 144°C.

Example 5

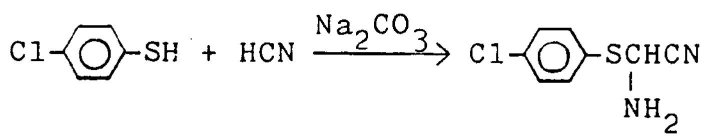


(Compound NO. 25)

In a 100ml flask were placed p-chlorothiophenol (2.9g), 20% sodium carbonate (10.6g) and acetonitrile (20ml). Liquid hydrogen cyanide (3.3g) was added to this solution at 10°C and stirring was continued at 20°C for 3 hours. The reaction mixture was poured into 10% sodium hydroxide (30g) which was precooled at 5°C in 300ml glass vessel, followed by extracting with chloroform (20ml) twice.

The chloroform layer was dried over anhydrous magnesium sulfate. The residue left after the evaporation of chlorofolm was purified by column chromatography. p-Chlorophenylthioamino acetonitrile (1.7g) was obtained in a yield of 42.9%. m.p. 74 - 76°C.

Example 6

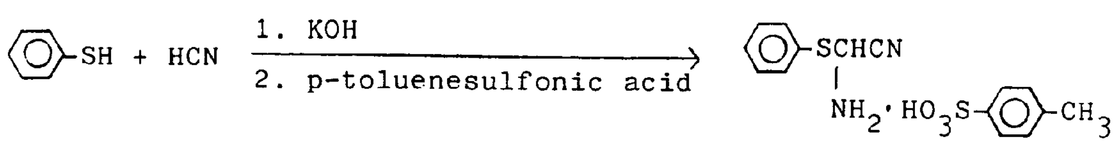


(Compound NO. 1)

In a 100ml flask were placed thiophenol (2.2g), potassium hydroxide (0. 6g) and ethyl alcohol (30ml ).

Liquid hydrogen cyanide (3.3g) was added to this solution at 5°C and stirring was continued at 20 °C for 2 hours. The reaction mixture was poured into 10% sodium hydroxide (30g) which was precooled at 5°C in 300ml glass vessel, followed by extracting with chloroform (20ml) twice. The chloroform layer was dried over anhydrous magnesium sulfate.

When worked up as Example 2, phenylthioaminoacetonitrile p-toluenesulfonate (3.1g) was obtained in a yield of 45.5%.

Example 7

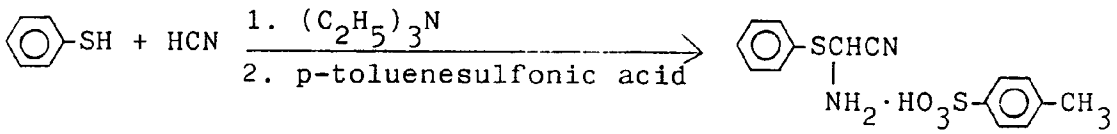


(Compound NO. 1)

In a 100ml flask were placed thiophenol (2.2g), triethylamine (1.0g) and ethylacetate (15ml).

Liquid hydrogen cyanide (3.3g) was added to this solution at 5°C and stirring was continued at 20°C for 2.5 hours. The reaction mixture was poured into 10% sodium hydroxide (30g) which was precooled at 5°C in 300ml glass vessel, followed by extracting with chloroform (20ml) twice. The chloroform layer was dried over anhydrous magnesium sulfate. When worked up as Example 2, phenylthioaminoacetonitrile p-toluenesulfonate (3.4g) was obtained in a yield of 51.0%.

Example 8

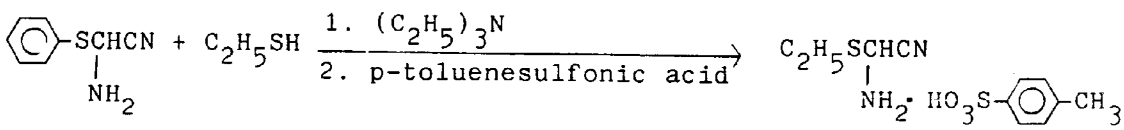


(Compound No. 4)

In a 100ml flask were placed phenylthioaminoacetonitrile (6.6g), triethylamine (0.8g) and acetonitrile (30ml). Ethylmercaptan (1.5g) was added to this solution at 15°C and stirring was continued at 20°C for 12 hours. To the residue left after the evaporation of acetonitrile and triethylamine, dichloromethane (10ml) and p-toluenesulfonic acid monohydrate (7.6g) were added. Then the reaction mixture stirred at 0 - 5°C for 10 minutes. The white precipitate was filtered, washed with diethyl ether (20ml) and acetonitrile (20ml) and dried in a vacuum desiccator.

Ethylthioaminoacetonitrile p-toluenesulfonate (9.4g) was obtained in a yield of 69.6%.

Inclusive of the above, each compound with the scope of the present invention which can be prepared in analogous method is tabulated in Table 1.

Table 1

| Compound NO. | Structure RSCHCN<br>\|<br>$NH_2$ R | salt | ( ) m.p.°C |
|---|---|---|---|
| 1 | 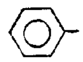 | 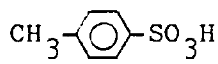 | ( 145 - 146 ) |
| 2 | 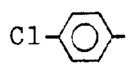 | " | ( 149 - 150 ) |
| 3 | 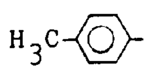 | " | ( 153 - 155 ) |
| 4 | $C_2H_5$- | " | ( 125 - 126 ) |
| 5 | 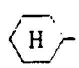 | " | ( 137 - 139 ) |
| 6 | $CH_3(CH_2)_7$- | " | ( 120 - 122 ) |
| 7 | $CH_2$=$CHCH_2$- | " | ( 127 - 129 ) |
| 8 | 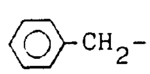 | " | ( 143 - 144 ) |

| | | | |
|---|---|---|---|
| 9 | 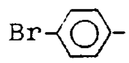 | " | ( 157 - 158.5) |
| 10 | 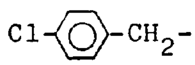 | " | ( 159 - 160.5) |
| 11 | 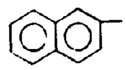 | " | ( 155 - 156.5) |
| 12 | 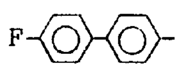 | " | ( 156 - 158 ) |
| 13 | 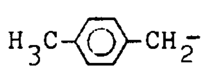 | " | ( 152 - 153 ) |
| 14 | 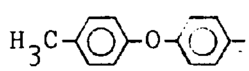 | " | ( 147 - 148 ) |
| 15 | 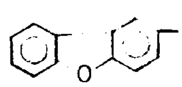 | " | ( 160 - 161 ) |
| 16 | 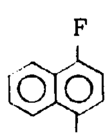 | " | ( 161 - 162 ) |
| 17 | 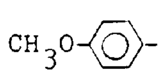 | " | ( 154 - 155 ) |

| | | | |
|---|---|---|---|
| 18 | $CH_3O\underset{\underset{O}{\|}}{C}CH_2CH_2-$ | " | ( 99 - 100 ) |
| 19 | $CH_3(CH_2)_{11}-$ | " | ( 119 - 120.5 ) |
| 20 | $CH_3-$ | " | ( 134 - 135 ) |
| 21 | 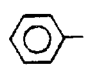 | HCl | ( 115 - 117 ) |
| 22 | " | $H_2SO_4$ | ( 84 - 85 ) |
| 23 | " | $(COOH)_2$ | ( 99 - 100 ) |
| 24 | " | — | $N_D^{20}$ 1.5955 |
| 25 | 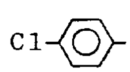 | — | ( 74 - 76 ) |
| 26 | 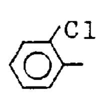 | 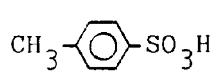 | ( 147 - 148 ) |

| | | | |
|---|---|---|---|
| 27 | 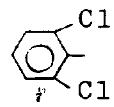 | " | |
| 28 | 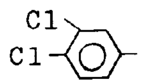 | " | ( 162 - 163 ) |
| 29 | 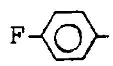 | " | ( 159 - 161 ) |
| 30 | 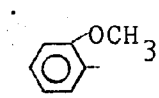 | " | |
| 31 | 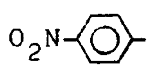 | " | |
| 32 | 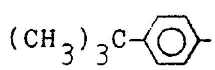 | — | $N_D^{26}$ 1.5608 |
| 33 | 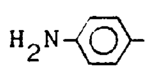 | 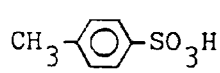 | ( 146 - 147 ) |
| 34 | 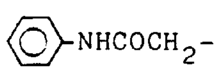 | " | ( 223 - 225 ) |
| 35 | 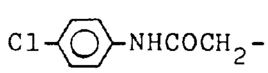 | " | |

| | | | |
|---|---|---|---|
| 36 | $CH_3(CH_2)_3NHCOCH_2-$ | " | |
| 37 | 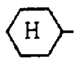$-CHCOCH_2-$ | " | |
| 38 | $CH_3(CH_2)_{11}NHCOCH_2$ | " | |
| 39 | 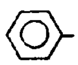$-CH_2NHCOCH_2-$ | " | |
| 40 | $HOCH_2CH_2-$ | " | |
| 41 | $ClCH_2CH_2CH_2-$ | " | ( 140 - 141 ) |
| 42 | $CH_3(CH_2)_3-$ | " | ( 145 - 146 ) |
| 43 | $CH_3CH_2CH_2-$ | " | ( 138 - 139 ) |
| 44 | $CH_3CO-$ | " | |

| | | | |
|---|---|---|---|
| 45 | 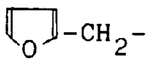 | " | ( 136 - 138 ) |
| 46 | 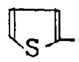 | " | ( 153 - 155 ) |
| 47 | 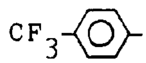 | " | ( 149 - 151 ) |
| 48 | 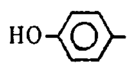 | " | ( 164 - 165 ) |
| 49 | 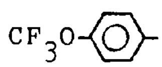 | " | |
| 50 | $HSCH_2CH_2-$ | " | ( 120 - 122 ) |
| 51 | 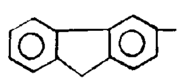 | " | ( 159 - 161 ) |
| 52 | 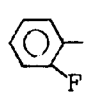 | " | ( 130 - 131 ) |
| 53 | 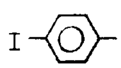 | " | ( 152 - 153 ) |

| | | | |
|---|---|---|---|
| 54 | 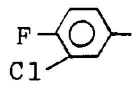 | " | ( 150 - 151 ) |
| 55 | 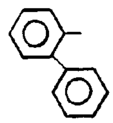 | " | ( 119 - 120 ) |
| 56 | 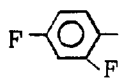 | " | ( 153 - 155 ) |
| 57 | 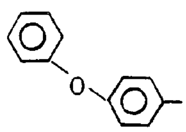 | " | ( 123 - 125 ) |
| 58 | 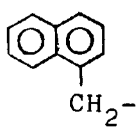 | " | ( 145 - 147 ) |
| 59 | 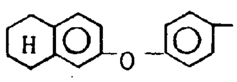 | " | ( 150 - 151 ) |
| 60 | 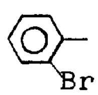 | " | ( 144 - 145 ) |
| 61 | 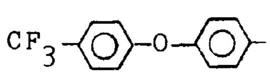 | " | ( 158 - 160 ) |
| 62 | 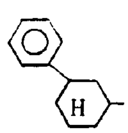 | " | ( 130 - 132 ) |

| | | | |
|---|---|---|---|
| 63 | 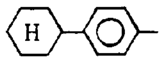 | " | ( 148 - 149 ) |
| 64 | 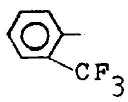 | " | ( 138 - 139 ) |
| 65 | 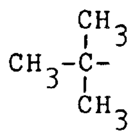 | — | $N_D^{26}$ 1.4928 |
| 66 | " | $(COOH)_2$ | ( 111 - 112 ) |
| 67 | 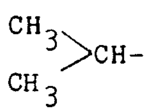 | — | $N_D^{26}$ 1.5586 |
| 68 | 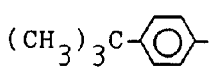 | $(COOH)_2$ | ( 99 - 100 ) |
| | | | |
| | | | |
| | | | |

Industrial Applicability:

The aminoacetonitrile derivatives of this invention, which are compounds substituted at the 2-position with RS- group, can be isolated stably. The derivatives are very reactive and react with various reagents to give useful products, being useful as synthetic materials for intermediates of such as pharmaceutical, agricultural chemicals, dyes, perfumes and polymers. In addition, the derivatives can be synthesized directly from hydrogen cyanide under mild conditions as described in the aforementioned examples and thus prepared at a low cost, having extremely great industrial significance.

## Claims

1. An aminoacetonitrile derivative or salts thereof having the fomula

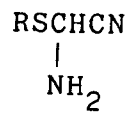

wherein R represents (a) an alkyl group which may be substituted by phenyl group(s) (which may be substituted by halogen atom(s) or $C_{1-3}$ alkyl group(s)), $C_{1-3}$ alkoxycarbonyl group(s), hydroxy group(s), mercapto group(s) halogen atom(s), naphthyl group(s), furyl group(s) or $R_1$NHCO-group(s) (where $R_1$ is a phenyl group which may be substituted by halogen atom(s), an alkyl group which may be substituted by phenyl group(s) or a cycloalkyl group), (b) a phenyl group which may be substituted by halogen atom(s), $C_{1-5}$ alkyl group(s) (which may be substituted by halogen atom(s), $C_{1-5}$ alkyl group(s) (which may be substituted by halogen atom(s) or phenyl group(s)), cylcoalkyl group(s) phenyl group(s) (which may be substituted by halogen atom(s)), phenoxy group(s) (which may be substituted by $C_{1-3}$ alkyl group(s) or haloalkyl group(s)), tetrahyronaphthoxy group(s), $C_{1-3}$ alkoxy group(s) (which may be substituted halogen atom(s)), nitro group(s), amino group(s) or hydroxy group(s), (c) a cycloalkyl group, (d) a $C_{2-4}$ alkyl group, (e) a naphthyl group which may be substituted by halogen atom(s), (f) a dibenzofuranyl group, (g) a $C_{1-3}$ alkylcarbonyl group, (h) a thienyl group or a (e) a fluorenyl group.

2. A process for the preparation of an aminoacetonitrile derivative or salts thereof having the formula

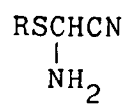

which comprises reacting hydrogen cyanide with a thiol compound having the formula RSH wherein R represents as defined above.

3. A process for the preparation of an aminoacetonitrile derivative or salts thereof having the formula

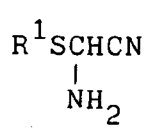

which comprises reacting a compound or salts thereof having the formula

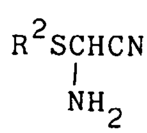

with a thiol compound having the formula

$R^1SH$

wherein $R^1$ represents (a) an alkyl group which may be substituted by phenyl group(s) (which may be substituted by halogen atom(s) or $C_{1-3}$ alkyl group(s)), $C_{1-3}$ alkoxy-carbonyl group(s), hydroxy group(s), mercapto group(s), halogen atom(s), naphthyl group(s), furyl group(s) or $R_1$NHCO-group(s) (where $R_1$ is a phenyl group which may be substituted by halogen atom(s), an alkyl group which may be substituted by phenyl group(s) or a cycloalkyl group), (b) a cycloalkyl group, or (c) a $C_{2-4}$ alkyl group ;

$R^2$ represents (a) a phenyl group which may be substituted by halogen atom(s), $C_{1-5}$ alkyl group(s) (which may be substituted by halogen atom(s) or phenyl group(s)), cycloalkyl group(s), phenyl group(s) (which may be substituted by halogen atom(s)), phenoxy group(s) (which may be substituted by $C_{1-3}$ alkyl group(s) or haloalkyl group(s)), tetrahydronaphthoxy group(s), $C_{1-3}$ alkoxy group(s) (which may be substituted halogen atom(s)), nitro group(s), amino group(s) or hydroxy group(s), (b) a naphthyl group which may be substituted by
halogen atoms, (c) a thienyl group or (d) a fluorenyl group.

## Patentansprüche

1. Ein Aminoacetonitril-Derivat oder Salze davon der Formel

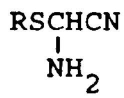

in der R
(a) eine Alkylgruppe, die durch (eine) Phenylgruppe(n) substituiert sein kann, (die durch (ein) Halogenatom(e) oder (eine) $C_{1-3}$-Alkylgruppe(n) substituiert sein kann/können), (eine) $C_{1-3}$-Alkoxycarbonylgruppe(n), (eine) Hydroxygruppe(n), (eine) Mercaptogruppe(n), (ein) Halogenatom(e), (eine) Naphthylgruppe(n), (eine) Furylgruppe(n) oder (eine) $R_1$NHCO-Gruppe(n), (wobei $R_1$ eine Phenylgruppe ist, die durch (ein) Halogenatom(e) substituiert sein kann), eine Alkylgruppe, die durch (eine) Phenylgruppe(n) oder eine Cycloalkylgruppe substituiert sein kann),
(b) eine Phenylgruppe, die substituiert sein kann durch (ein) Halogenatom(e), (eine) $C_{1-5}$ -Alkylgruppe(n), (die durch (ein) Halogenatom(e) oder (eine) Phenylgruppe(n) substituiert sein kann), (eine) Cycloalkylgruppe(n), (eine) Phenylgruppe(n), (die durch (ein) Halogenatom(e) substituiert sein kann), (eine) Phenoxygruppe(n), (die durch (eine) $C_{1-3}$-Alkylgruppe(n) oder (eine) Halogenalkylgruppe(n) substituiert sein kann), (eine) Tetrahydronaphthoxygruppe(n), (eine) $C_{1-3}$-Alkoxygruppe(n), (die durch (ein) Halogenatom(e) substituiert sein kann), (eine) Nitrogruppe(n), (eine) Aminogruppe(n) oder (eine) Hydroxygruppe(n),
(c) eine Cycloalkylgruppe,
(d) eine $C_{2-4}$-Alkenylgruppe,
(e) eine Naphthylgruppe, die durch (ein) Halogenatom(e) substituiert sein kann,
(f) eine Dibenzofuranylgruppe,
(g) eine $C_{1-3}$-Alkylcarbonylgruppe,
(h) eine Thienylgruppe oder
(i) eine Fluorphenylgruppe
bedeutet.

2. Verfahren zur Herstellung eines Aminoacetonitrile-Derivats oder von Salzen davon der Formel

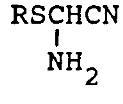

umfassend das Umsetzen von Cyanwasserstoff mit einer Thiolverbindung der Formel RSH, wobei R wie oben definiert ist.

3. Verfahren zur Herstellung eines Aminoacetonitril-Derivats oder von Salzen davon der Formel

$$R^1SCHCN$$
$$|$$
$$NH_2$$

umfassend das Umsetzen einer Verbindung oder von Salzen davon entsprechend der Formel

$$R^2SCHCN$$
$$|$$
$$NH_2$$

mit einer Thiolverbindung der Formel $R^1SH$,
in der $R^1$

(a) eine Alkylgruppe, die durch (eine) Phenylgruppe(n) substituiert sein kann, (die durch (ein) Halogenatom(e) oder (eine) $C_{1-3}$-Alkylgruppe(n) substituiert sein kann/können), (eine) $C_{1-3}$-Alkoxycarbonylgruppe(n), (eine) Hydroxygruppe(n), (eine) Mercaptogruppe(n), (ein) Halogenatom(e), (eine) Naphthylgruppe(n), (eine) Furylgruppe(n) oder (eine) $R_1$NHCO-Gruppe(n), (wobei $R_1$ eine Phenylgruppe ist, die durch (ein) Halogenatom(e) substituiert sein kann), eine Alkylgruppe, die durch (eine) Phenylgruppe(n) oder eine Cycloalkylgruppe substituiert sein kann),
(b) eine Cycloalkylgruppe oder
(c) eine $C_{2-4}$-Alkenylgruppe,
bedeutet,

$R^2$

(a) eine Phenylgruppe, die substituiert sein kann durch (ein) Halogenatom(e), (eine) $C_{1-5}$ -Alkylgruppe(n), (die durch (ein) Halogenatom(e) oder (eine) Phenylgruppe(n) substituiert sein kann), (eine) Cycloalkylgruppe(n), (eine) Phenylgruppe(n), (die durch (ein) Halogenatom(e) substituiert sein kann), (eine) Phenoxygruppe(n), (die durch (eine) $C_{1-3}$-Alkylgruppe(n) oder (eine) Halogenalkylgruppe(n) substituiert sein kann), (eine) Tetrahydronaphthoxygruppe(n), (eine) $C_{1-3}$-Alkoxygruppe(n), (die durch (ein) Halogenatom(e) substituiert sein kann), (eine) Nitrogruppe(n), (eine) Aminogruppe(n) oder (eine) Hydroxygruppe(n),
(b) eine Naphthylgruppe, die substituiert sein kann durch Halogenatome,
(c) eine Thienylgruppe oder
(d) eine Fluorenylgruppe
bedeutet.

**Revendications**

**1.** Derivé d'aminoacétonitrile ou ses sels ayant pour formule

$$RSCH\,CN$$
$$|$$
$$NH_2$$

où R représente (a) un groupe alkyle qui peut être substitué par un ou plusieurs groupes phényles (qui peuvent être substitué par un ou plusieurs atomes d'halogène ou un ou plusieurs groupes alkyles $C_{1-3}$), un ou plusieurs groupes alcoxycarbonyles $C_{1-3}$, un ou plusieurs groupes hydroxy, un ou plusieurs groupes mercapto, un ou plusieurs atomes d'halogène, un ou plusieurs groupes naphtyles, un ou plusieurs groupes furyles, ou plusieurs groupes $R_1$NHCO (où $R_1$ est un groupe phényle qui peut être substitué par un ou plusieurs atomes d'halogène, un groupe alkyle qui peut être substitué par un ou plusieurs groupes phényles ou un groupe cycloalkyle), (b) un groupe phényle qui peut être substitué par un ou plusieurs atomes d'halogène, un ou plusieurs groupes alkyles $C_{1-5}$ (qui peuvent être substitués par un ou plusieurs atomes d'halogène ou un ou plusieurs groupes phényles), un ou plusieurs groupes cycloalkyles, un ou plusieurs groupes phényles (qui peuvent être substitué par un ou plusieurs atomes d'halogène), un ou plusieurs

groupes phénoxy (qui peuvent être substitués par un ou plusieurs groupes alkyles $C_{1-3}$ ou un ou plusieurs groupes haloalkyles), un ou plusieurs groupes tétrahydronaphtoxy, un ou plusieurs groupes alcoxy $C_{1-3}$ (qui peuvent être substitués par un ou plusieurs atomes d'halogène), un ou plusieurs groupes nitro, un ou plusieurs groupes amino ou un ou plusieurs groupes hydroxy, (c) un groupe cycloalkyle (d) un groupe alkényle $C_{2-4}$, (e) un groupe naphtyle qui peut être substitué par un ou plusieurs atomes d'halogène, (f) un groupe dibenzofuranyle, (g) un groupe alkylcabonyle $C_{1-3}$, (h) un groupe thiényle ou (i) un groupe fluorényle.

**2.** Procédé pour la préparation d'un dérivé d'aminoacétonitrile ou ses sels ayant pour formule

$$\begin{array}{l} RSCHCN \\ \quad\;\; | \\ \quad NH_2 \end{array}$$

qui comprend la réaction de cyanure d'hydrogène avec un composé thiol ayant pour formule RSH, où R représente ce qui est défini ci-dessus.

**3.** Procédé pour la préparation d'un dérivé d'aminoacétronitrile ou ses sels ayant pour formule

$$\begin{array}{l} R^1SCHCN \\ \quad\;\; | \\ \quad NH_2 \end{array}$$

qui comprend la réaction d'un composé ou ses sels ayant pour formule

$$\begin{array}{l} R^2SCHCN \\ \quad\;\; | \\ \quad NH_2 \end{array}$$

avec un composé thiol ayant pour formule

$$R^1SH$$

où $R^1$ représente (a) un groupe alkyle qui peut être substitué par un ou plusieurs groupes phényles (qui peuvent être substitués par un ou plusieurs atomes d'halogène ou un ou plusieurs groupes alkyles $C_{1-3}$), un ou plusieurs groupes alkcoxycarbonyles $C_{1-3}$, un ou plusieurs groupes hydroxy, un ou plusieurs groupes mercapto, un ou plusieurs atomes d'halogène, un ou plusieurs groupes naphtyles, un ou plusieurs groupes furyles ou un ou plusieurs groupes $R_1$NHCO (où $R_1$ est un groupe phényle qui peut être substitué par un ou plusieurs atomes d'halogène, un groupe alkyle qui peut être substitué par un ou plusieurs groupes phényles ou un groupe cylcoalkyle), (b) un groupe cycloalkyle ou (c) un groupe alkényle $C_{2-4}$;
$R^2$ représente (a) un groupe phényle qui peut être substitué par un ou plusieurs atomes d'halogène, un ou plusieurs groupes alkyles $C_{1-5}$ (qui peuvent être substitués par un ou plusieurs atomes d'halogène ou un ou plusieurs groupes phényles), un ou plusieurs groupes cycloalkyles, un ou plusieurs groupes phényles (qui peuvent être substitués par un ou plusieurs atomes d'halogène, un ou plusieurs groupes phénoxy (qui peuvent être substitués par un ou plusieurs groupes alkyles $C_{1-3}$ ou un ou plusieurs groupes haloalkyles), un ou plusieurs groupes tétrahydronaphtoxy, un ou plusieurs groupes alcoxy $C_{1-3}$ (qui peuvent être substitués par un ou plusieurs atomes d'halogène), un ou plusieurs groupes nitro, un ou plusieurs groupes amino, ou un ou plusieurs groupes hydroxy, (b) un groupe naphtyle qui peut être substitué par des atomes d'halogène, (c) un groupe thiényle ou (d) un groupe fluorényle.